# EUROPEAN PATENT SPECIFICATION

(11) **EP 2 768 478 B1**
(45) Date of publication and mention of the grant of the patent: **17.03.2021**
(21) Application number: 12779131.7
(22) Date of filing: 16.10.2012
(51) Int. Cl.: A61K 9/00, A61K 31/5415, A61K 47/10, A61K 47/36, A61K 47/38, A61P 29/00, A61K 47/26, A61K 9/06

(54) **PASTE COMPRISING THE NON-STEROIDAL ANTI-INFLAMMATORY DRUG MELOXICAM**
PASTE MIT DEM NICHTSTEROIDALEN ANTIRHEUMATIKUM MELOXICAM
PÂTE AVEC L'ANTI-INFLAMMATOIRE NON STEROÏDIEN MÉLOXICAM

(30) Priority: 17.10.2011 GB 201117858
(43) Date of publication of application: 27.08.2014
(73) Proprietor: Norbrook Laboratories Limited, Newry County Down BT35 6PU (GB)
(72) Inventor: REYNOLDS, Louise, Newry County Down BT35 6PU (GB); BLAKELY, William, Newry County Down BT35 6PU (GB); UMRETHIA, Manish, Newry County Down BT35 6PU (GB)
(74) Representative: HGF
(86) International application number: PCT/GB2012/000790
(87) International publication number: WO 2013/057461

(56) References cited:
- EP-A1- 1 582 221
- WO-A1-99/39713
- WO-A1-99/62498
- WO-A1-03/103691
- WO-A1-2004/026313
- WO-A1-2006/061351
- GB-A- 2 438 287
- US-A1- 2002 068 718
- US-A1- 2008 027 011
- US-B1- 6 423 697
- JANTHARAPRAPAP ET AL: "Effects of penetration enhancers on in vitro permeability of meloxicam gels", INTERNATIONAL JOURNAL OF PHARMACEUTICS, ELSEVIER BV, NL, vol. 343, no. 1-2, 30 August 2007 (2007-08-30), pages 26-33, XP022223248, ISSN: 0378-5173, DOI: 10.1016/J.IJPHARM.2007.04.011
- CHANG ET AL: "Formulation optimization of meloxicam sodium gel using response surface methodology", INTERNATIONAL JOURNAL OF PHARMACEUTICS, ELSEVIER BV, NL, vol. 338, no. 1-2, 23 May 2007 (2007-05-23), pages 48-54, XP022093436, ISSN: 0378-5173, DOI: 10.1016/J.IJPHARM.2007.01.033

## Description

The present invention relates to oral pastes comprising the non-steroidal anti-inflammatory drug meloxicam, a method of their production and their use in the alleviation of inflammation and pain in both acute and chronic musculoskeletal disorders in animals, especially horses.

Numerous pharmaceutically acceptable oral dosage forms, such as liquid suspensions, are known in the art. Such suspensions are liquid systems having solid particles dispersed substantially throughout.

EP-A-1,066,029 (Metacam™) describes a suspension for non-steroidal anti-inflammatory drugs that comprises about 0.1 to about 5% by weight of a highly dispersed silicon dioxide and about 0.05 to about 2% by weight of a hydrophilic polymer.

EP-A-1,520,578 further describes another suspension system for pharmaceuticals that comprises xanthan gum, a swelling agent, such as pregelatinised starch, a surfactant such as polyoxyethylene sorbitan monoleate, an amino polycarboxylic acid or salt thereof such as ethylenediaminetetraacetic acid (EDTA), and optionally a nucleation inhibitor, such as polyvinylpyrrolidone. Taste modifying agents, such as sugars, artificial sweetener, flavouring agents and mixtures thereof, can also be present and generally comprise 25 to 50 % by weight of the total composition.

WO-A-2006/061351 further describes a suspension comprising meloxicam suspended in an aqueous glycerol mixture, a thickening agent, one or more taste modifying agents and a buffer system for maintaining the pH in a range from 2 to 4, wherein the suspension is free or essentially free of silicon dioxide.

A common problem associated with the oral dosage forms, such as liquid suspensions, is that the suspensions are runny which result in them being easily split when administered to animals, especially horses, and that they have a high sedimentation rate.

Such problems result in an inaccuracy of the dose administered to the animal and an inefficient method of administering an active ingredient to an animal. To compensate for the loss of active ingredient due to separation and sedimentation the person administering the dose to the animal would need to administer another partial dose to ensure that a sufficient amount of active ingredient is administered. It would, however, be difficult to calculate the exact amount of active ingredient required and too much may, therefore, be ultimately administered to the animal. This is dangerous as several active ingredients, such as meloxicam, are toxic and, therefore; any over dosing could, therefore, be potentially fatal.

There is, therefore, a need to provide an oral dosage form which is efficient, can be accurately administered to an animal and is, therefore, safe to use.

It has now been surprisingly found that an oral dosage form in the form of a paste overcomes the problems associated with the known liquid suspensions as the paste does not separate in use and sedimentation does not occur. Paste formulations are also advantageous in that they are ready to use and do not require to be shaken/mixed before use, in contrast to known liquid suspensions.

Hence according to the present invention there is provided an oral paste comprising:
(i) meloxicam,
(ii) a viscosity modifying agent in an amount of from 3 to 10% w/v of the paste,
(iii) a liquid vehicle, wherein the viscosity modifying agent is a mixture of hydroxypropylcellulose and xanthan gum.

A process for the manufacture of the paste, comprising the following steps:
a) adding hydroxypropylcellulose and xanthan gum to a liquid vehicle and mixing until a homogeneous gel is made; and
b) adding meloxicam to the homogeneous gel of step a) whilst stirring to obtain a paste;
wherein hydroxypropylcellulose and xanthan gum are present in an amount of from 3 to 10% w/v of the paste.

The present invention provides said oral paste for use in alleviating pain and inflammation in both acute and chronic musculoskeletal disorders in animals.

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

The oral pharmaceutical composition of the present invention is in the form of a paste.

A paste is a two-component semi-solid in which a drug, in this instance a non-steroidal anti-inflammatory drug, is dispersed as a powder in a liquid vehicle. The paste preferably has a viscosity of from about 7000 to about 15000 cps as measured by a Brookfield viscometer, spindle size 18 at a speed of 1 RPM and a temperature of 25°C.

The liquid vehicle can be an aqueous or fatty base, for example. The liquid vehicle containing the non-steroidal anti-inflammatory drug can be selected from the group consisting of water, a polyhydroxy liquid such as glycerin, propylene glycol, or polyethylene glycol, a vegetable oil or a mineral oil, including mixtures of two or more thereof. In a preferred'embodiment the liquid vehicle is water. The particle size of the active ingredient in the paste can be as large as 100 µm.

One or more non-steroidal anti-inflammatory drugs can be selected from the group comprising of salicylates such as aspirin, methyl salicylate, Diflunisal and amoxiprin; acetaminophen; arylalkanoic acids such as diclofenac, indomethacin and sulindac; propionic acid derivatives (profens) such as ibuprofen, carprofen, naproxen and ketoprofen; N-Arylanthranlic acids (fenamic acid derivatives) such as mefanamic acid, meclofenamic acid and flufenamic acid; oxicams such as piroxicam, sudoxicam, isoxicam and meloxicam; coxibs such as celecoxib, rofecoxib, valdecoxib, parecoxib and etoricoxib; sulphonanilides such as nimesulide; and non-steroidal anti-inflammatory drugs that have both cyclo-oxygenase (II) and lipooxygenase inhibition properties such as tepoxalin and mixtures of two or more thereof.

The amount of non-steroidal anti-inflammatory drug present in the paste should be sufficient to provide a therapeutic amount of the active and a convenient dosage unit. Accordingly, the at least one non-steroidal anti-inflammatory drug can be present in an amount of from about 1 to about 10% w/v of the paste, preferably about 3 to about 8% w/v of the paste, even more preferably about 4% to about 6% w/v of the paste, and most preferably in an amount of about 5% w/v of the paste.

In a preferred embodiment the non-steroidal anti-inflammatory drug is present in both micronised and unmicronised form.

The non-steroidal anti-inflammatory drug in its micronised form will have a D_{50%} (diameter of 50% of particles) of less than about 10 microns, for example from about 1 to about 10 microns, preferably less than about 5 microns.

The non-steroidal anti-inflammatory drug in its unmicronised form will have a D_{50%} (diameter of 50% of particles) of greater than about 10 microns, for example from about 10 to about 200 microns, preferably less than abou 100 microns.

The particle size is measured by laser diffraction. The particles of the non-steroidal anti-inflammatory drug having a D_{50%} (diameter of 50% of particles) of less than 10 microns . can be obtained for example by micronisation or by milling. Preferably the particles are obtained by micronisation.

In a preferred embodiment at least about 60% of the total amount of the non-steroidal anti-inflammatory drug, more preferably between about 70 to about 80% of the particles, and most preferably at least about 74% is micronised. In one embodiment all of the non-steroidal anti-inflammatory drug can be present in the micronised form.

In a preferred embodiment about 40% of the total amount of the non-steroidal anti-inflammatory drug, more preferably between about 25 to about 35% of the particles, and most preferably at least about 26% is unmicronised. In one embodiment all of the non-steroidal anti-inflammatory drug can be present in unmicronised form.

In a preferred embodiment the micronised form of the non-steroidal anti-inflammatory drug is present in an amount of at about 74% of the total amount of non-steroidal anti-inflammatory drug present and the non-steroidal anti-inflammatory drug in its unmicronised form is present in an amount of about 26% of the total amount of non-steroidal anti-inflammatory drug present.

The non-steroidal anti-inflammatory drug can also be used in combination with other drugs such as but not limited to antimicrobials, antibiotics, antivirals, anti-ulcer/anti-acid agents and anti-cancer agents or a combination of two or more thereof.

The non-steroidal anti-inflammatory drug(s) are present in a "unit dose volume" of the paste in a therapeutically effective amount, which is in an amount that produces the desired therapeutic response upon oral administration. In determining such amounts, the particular non-steroidal anti-inflammatory drug(s) being administered, the bioavailability characteristics of the non-steroidal anti-inflammatory drug , the dose regime, the age and weight of the recipient, and other factors must be considered, as known in the art. As used herein a "unit dose volume" of the paste is a convenient volume for dosing the product to a recipient. The dosing directions instruct the recipient to take amounts that are multiples of the unit dose depending on for example the age or weight of the recipient. Typically the unit dose volume of the paste will contain an amount of non-steroidal anti-inflammatory drug that is therapeutically effective for the smallest patient. An effective amount may be achieved by multiple dosing.

The paste is administered in such an amount that a single daily dosage comprises from about 0.05 mg to about 1.0 mg of non-steroidal anti-inflammatory drug/kg body weight. In a preferred embodiment a typical once daily dosage ranges from about 0.5 to about 0.7 mg, and most preferably in an amount of about 0.6 mg non-steroidal anti-inflammatory drug/kg body weight.

The paste is to be administered directly into the mouth of the animal. Dosing of the paste can be done using an appropriate metering system such as, for example a calibrated syringe, or a pre-filled dispenser that can deliver calibrated amounts of the paste.

In a preferred embodiment the paste is administered directly into the mouth of the animal, once daily for up to 14 days.

The degree of cohesiveness, plasticity, and syringeability of pastes is attributed to the viscosity modifying agent.

Examples of viscosity modifying agents include hydroxypropylcellulose, methylcellulose, hydroxyethylcellulose, hydroxypropylmethyl cellulose, hydroxyethylpropyl celluose, polyethylene glycol, propylene glycol, starches, such as maize or corn starch, potato starch, rice starch, tapioca starch and wheat starch, hydroxypropyl starch, carboxyvinyl polymers, carbomers such as Carbopol, carboxymethyl cellulose and salts thereof, microcrystalline cellulose, agar, polyvinyl alcohol, alginic acid, potassium alginate, polyvinyl pyrrolidone, carmellose sodium, maltodextrin, dextrin, gelatin, pectin, poloxamer, polycarbophil, pregelatinised starch, polysaccharide gums such as guar, acacia, gellan, carrageenan, xanthan and tragacanth gums and mixtures of two or more thereof.

Xanthan gum and hydroxypropylcellulose are present as viscosity modifying agents.

Examples of suitable xanthan gums that can be used include Rhodigel 80™, Rhodigel 23™, Keltrol™, Keltrol™ F, Keltrol™ T, Keltrol™ TF, Keltrol™ 1000 and Merezan™. Rhodigel 23™ is preferred.

The viscosity modifying agents are present: in an amount of from 3 to 10% w/v of the paste and preferably present in an amount of about 4 % w/v of the paste.

In a preferred embodiment the xanthan gum is present in an amount of from about 0.1 to about 5% w/v of the paste, preferably in an amount of from about 0.5 to about 2% w/v of the paste, and most preferably in an amount of about 0.8% w/v of the paste and the hydroxypropyl cellulose is present in an amount of from about 1 to about 8 % w/v of the paste, preferably in an amount of from about 2 to about 5% w/v of the paste, and most preferably in an amount of about 3% w/v of the paste.

The paste may also contain a wetting agent and/or a stabiliser.

Wetting agents may be surfactants, hydrophilic colloids or solvents.

Surfactants can be ionic, non-ionic, cationic and amphiphilic surfactants. Examples of suitable ionic and non-ionic surfactants include sodium lauryl sulphate, sulphate, docusate sodium, polysorbate 80, poloxamers and povidones. An example of a suitable cationic surfactant is cetyltrimethyl ammoinum bromide.Examples of suitable amphiphilic surfactants include tweens, pluronics, cremophor and solutol.

Examples of suitable hydrophilic colloids include acacia, tragacanth, alginates, guar gum, pectin, gelatin, wool fat, egg yolk, bentonite, Veegum and methylcellulose.

Examples of suitable solvents include alcohol, glycerol, polyethylene glycol and polypropylene glycol.

In a preferred embodiment the wetting agent is selected from the group consisting of glycerol, polysorbate 80, polyethylene glycol and mixtures of two or more thereof. Most preferably the wetting agent is glycerol.

Wetting agents can be present in an amount of from about 0 to about 50% w/v of the paste, preferably in an amount of from about 5 to about 30% w/v of the paste, more preferably in an amount of from about 10 to about 20% w/v of the paste and most preferably in an amount of about 15% w/v of the paste.

Examples of stabilisers which may be present include natural gums, cellulose derivatives, and the surfactants and solvents as detailed above as suitable wetting agents.

Stabilisers can be present in an amount of from about 0 to about 50% w/v of the paste, preferably in an amount of from about 5 to about 30% w/v of the paste and more preferably in an amount of from about 10 to about 20% w/v of the paste.

In a preferred embodiment the stabiliser is selected from the group consisting of glycerol, polysorbate 80, polyethylene glycol and mixtures of two or more thereof. Most preferably the stabiliser is glycerol.

In a preferred embodiment the paste includes glycerol, polysorbate 80 or polyethylene glycol as a wetting agent and a stabiliser. When the component acts as both the wetting agent and stabiliser it can be present in an amount of from about 0.5 to about 50% w/v of the paste, preferably from about 10 to about 30% w/v of the paste, and most preferably in an amount of about 15% w/v of the paste.

The pastes may also contain one or more of the following additives: preservatives, taste modifying agents such as flavouring agents, bulk sweeteners, intense sweeteners, colourings and humectants.

In order to protect the oral pastes of the present invention from microbial contamination during use, a preservative can be added. This preservative is preferably selected from the group consisting of benzoic acid and the sodium or potassium salts thereof, scorbic acid and the sodium or potassium salts thereof, benzyl alcohol, methyl alcohol, phenolic compounds such as phenol, quaternary compounds such as benzalkonium chloride, methyl parahydroxybenzoate, ethyl para-hydroxybenzoate, propyl parahydroxybenzoate, butyl parahydroxybenzoate, parabens such as methyl, ethyl, propyl and butyl and mixtures of two or more thereof. In a preferred embodiment the preservative is benzyl alcohol.

The preservative can be present in an amount of from about 0.02 to about 5% w/v of the paste, preferably about 0.1 to about 3% w/v of the paste and most preferably in an amount of about 1% w/v of the paste.

Examples of bulk sweeteners are sorbitol, mannitol, fructose, sucrose, maltose, isomalt, glucose, hydrogenated glucose syrup and xylitol, and mixtures of two or more thereof. In a preferred embodiment the bulk sweetener is sorbitol.

The bulk sweetener can be present in an amount of from about 1 to about 10% w/v of the paste, preferably about 3 to about 6% w/v of the paste, more preferably about 5 to about 6% w/v of the paste and most preferably in an amount of about 5.6% w/v of the paste.

Examples of intense sweeteners are saccharin, aspartame, acesulfame, cyclamate, alitame, taumatin, a dihydrochalcone sweetener, monellin, neohesperidin, neotame, stevioside and sucralose, the pharmaceutically acceptable salts thereof such as sodium or calcium saccharin, acesulfame potassium or sodium cyclamate, and mixtures of two or more thereof. In a preferred embodiment the intense sweetener is sodium saccharin.

The intense sweetener can be present in an amount of from about 0.01 to about 1% w/v of the paste, preferably about 0.03 to about 0.08% w/v of the paste, more preferably about 0.05 to about 0.07% w/v of the paste, and most preferably in an amount of about 0.07% w/v of the paste.

Examples of flavouring agents are apple, cherry, raspberry, blackcurrant, strawberry flavour, honey, chocolate flavour and mint cool flavour. In a preferred embodiment the flavouring agent is apple.

Flavouring agents can be present in an amount of from 0.01 to about 3% w/v of the paste, preferably about 0.1 to about 1% w/v of the paste, and most preferably about 0.3% w/v of the paste.

Colouring agents may also be incorporated in the paste to provide an appealing colour to the paste. The colouring agents should be selected to avoid chemical incompatibilities with the other ingredients in the paste. Examples of suitable colouring agents include FD&C Red #40, FD&C Blue #1 and FD&C Red #33.

Colouring agents can be present in an amount of from about 0 to about 0.1% w/v of the paste, preferably about 0 to about 0.0001% w/v of the paste, and most preferably about 0 to about 0.001% w/v of the paste.

Humectants are used to prevent the paste that may collect at the nozzle of a dispenser from forming a hard crust. Examples of humectants include glycerin and propylene glycol.

Humectants can be present in an amount of from about 0 to about 20% w/v of the paste, preferably about 0 to about 5% w/v of the paste, and most preferably about 0 to about 2% w/v of the paste.

In a preferred embodiment the paste is prepared by a) adding the viscosity modifying agent to a vehicle and mixing until a homogeneous gel is made; and
b) adding the non-steroidal anti-inflammatory drug to the homogeneous gel of step a) whilst stirring to obtain a paste.

The pastes can be used in the treatment of animals against disease. In particular the pastes can be used to alleviate inflammation and pain in both acute and chronic musculoskeletal disorders in animals, and in particular in horses.

The invention will now be described with respect to the following examples. The examples are not intended to be limiting of the scope of the present invention but read in conjunction with the detailed and general description above, provide further understanding of the present invention and an outline of the preferred processes for preparing the pastes of the invention.

### Example 1

The composition of the paste of the current invention is provided in Table 1.

| **Ingredient** | **% w/v** |
|---|---|
| Meloxicam (micronised) | 3.7 |
| Meloxicam (unmicronised) | 1.3 |
| Hydroxypropylcellulose | 3 .0 |
| Glycerol | 15.0 |
| Xanthan Gum | 0.8 |
| Apple flavour | 0.3 |
| Sodium Saccharin | 0.07 |
| Sorbitol | 5.6 |
| Benzyl alcohol | 1.0 |
| Purified water | to 100g |

The paste was prepared by adding the xanthan gum and hydroxypropyl cellulose into purified water and mixing together until a homogeneous gel was formed. Glycerol was added while stirring. Meloxicam was then added while stirring. Apple flavour, sodium saccharin, sorbitol and benzyl alcohol were then added while stirring.

The paste was administered directly into the mouth of the animal using a syringe. The unit dose volume was 0.6 mg meloxicam/kg body weight, once daily for up to 14 days.

The oral bioavailability was approximately 98%. Maximal plasma concentrations were obtained after approximately 4 hours. The accumulated factor of 1.08 suggests that meloxicam does not accumulate when administered daily.

Approximately 98% of meloxicam is bound to plasma proteins. The volume of distribution is 0.12 1/kg.

## Claims

1. An oral paste comprising:
(i) meloxicam,
(ii) a viscosity modifying agent in an amount of from 3 to 10% w/v of the paste,
(iii) a liquid vehicle, wherein the viscosity modifying agent is a mixture of hydroxypropylcellulose and xanthan gum

2. A paste according to claim 1 wherein meloxicam is present in an amount of from 3 to 8% w/v of the paste.

3. A paste according to any one of the preceding claims which further comprises a wetting agent and/or a stabiliser.

4. A paste according to claim 3 wherein the wetting agent and/or the stabiliser is glycerol.

5. A paste according to claim 4 wherein the glycerol is present in an amount of 15% w/v of the paste.

6. A paste according to any one of the preceding claims which further comprises a flavouring agent.

7. A paste according to any one of the preceding claims which further comprises a bulk sweetener.

8. A paste according to any one of the preceding claims which further comprises an intense sweetener.

9. A paste according to any one of the preceding claims which further comprises a preservative.

10. A paste according to any one of the preceding claims wherein the liquid vehicle is water.

11. A process for the manufacture of the paste as defined in any one of the preceding claims, comprising the following steps:
a) adding hydroxypropylcellulose and xanthan gum to a liquid vehicle and mixing until a homogeneous gel is made; and
b) adding meloxicam to the homogeneous gel of step a) whilst stirring to obtain a paste;
wherein hydroxypropylcellulose and xanthan gum are present in an amount of from 3 to 10% w/v of the paste.

12. The paste as defined in any one of claims 1 to 10 for use in alleviating pain and inflammation in both acute and chronic musculoskeletal disorders in animals.

## Patentansprüche

1. Orale Paste, umfassend:
(i) Meloxicam,
(ii) ein viskositätsmodifizierendes Mittel in einer Menge von 3 bis 10 % Gew./V. der Paste,
(iii) ein flüssiges Vehikel, wobei das viskositätsmodifizierende Mittel eine Mischung aus Hydroxypropylcellulose und Xanthangummi ist.

2. Paste nach Anspruch 1, wobei Meloxicam in einer Menge von 3 bis 8 % (Gew./V.) der Paste vorhanden ist.

3. Paste nach einem der vorhergehenden Ansprüche, die ferner ein Netzmittel und/oder einen Stabilisator umfasst.

4. Paste nach Anspruch 3, wobei das Benetzungsmittel und/oder der Stabilisator Glycerin ist.

5. Paste nach Anspruch 4, wobei das Glycerin in einer Menge von 15 % Gew./V. der Paste vorhanden ist.

6. Paste nach einem der vorhergehenden Ansprüche, die ferner einen Aromastoff umfasst.

7. Paste nach einem der vorangehenden Ansprüche, die ferner einen Massen-Süßstoff umfasst.

8. Paste nach einem der vorangehenden Ansprüche, die ferner einen intensiven Süßstoff umfasst.

9. Paste nach einem der vorangehenden Ansprüche, die ferner ein Konservierungsmittel umfasst.

10. Paste nach einem der vorangehenden Ansprüche, wobei das flüssige Vehikel Wasser ist.

11. Verfahren für die Herstellung der Paste nach einem der vorangehenden Ansprüche, umfassend die folgenden Schritte:
a) Zugeben von Hydroxypropylcellulose und Xanthangummi zu einem flüssigen Vehikel und Mischen, bis ein homogenes Gel hergestellt ist; und
b) Zugeben von Meloxicam zu dem homogenen Gel von Schritt a) unter Rühren, um eine Paste zu erhalten;
wobei Hydroxypropylcellulose und Xanthangummi in einer Menge von 3 bis 10 % Gew./V. der Paste vorhanden sind.

12. Paste nach einem der Ansprüche 1 bis 10 für die Verwendung bei der Linderung von Schmerzen und Entzündungen sowohl bei akuten als auch bei chronischen muskuloskelettalen Erkrankungen bei Tieren.

## Revendications

1. Pâte orale comprenant :
(i) du méloxicam,
(ii) un agent modifiant la viscosité en une quantité allant de 3 à 10 % en p/v de la pâte,
(iii) un véhicule liquide, ledit agent modifiant la viscosité étant un mélange d'hydroxypropylcellulose et de gomme de xanthane.

2. Pâte selon la revendication 1, ledit méloxicam étant présent en une quantité allant de 3 à 8 % en p/v de la pâte.

3. Pâte selon l'une quelconque des revendications précédentes, qui comprend en outre un agent mouillant et/ou un stabilisant.

4. Pâte selon la revendication 3, ledit agent mouillant et/ou ledit stabilisant étant le glycérol.

5. Pâte selon la revendication 4, ledit glycérol étant présent en une quantité de 15 % en p/v de la pâte.

6. Pâte selon l'une quelconque des revendications précédentes, qui comprend en outre un agent aromatisant.

7. Pâte selon l'une quelconque des revendications précédentes, qui comprend en outre un édulcorant brut.

8. Pâte selon l'une quelconque des revendications précédentes, qui comprend en outre un édulcorant intense.

9. Pâte selon l'une quelconque des revendications précédentes, qui comprend en outre un conservateur.

10. Pâte selon l'une quelconque des revendications précédentes, ledit véhicule liquide étant l'eau.

11. Procédé de fabrication de la pâte telle que définie dans l'une quelconque des revendications précédentes, comprenant les étapes suivantes :
a) l'ajout de l'hydroxypropylcellulose et de la gomme de xanthane à un véhicule liquide et le mélange jusqu'à ce qu'un gel homogène soit obtenu ; et
b) l'ajout du méloxicam au gel homogène de l'étape a) tout en agitant pour obtenir une pâte ;
ladite hydroxypropylcellulose et ladite gomme de xanthane étant présentes en une quantité allant de 3 à 10 % en p/v de la pâte.

12. Pâte telle que définie dans l'une quelconque des revendications 1 à 10 pour utilisation dans le soulagement de la douleur et de l'inflammation dans les troubles musculosquelettiques tant aigus que chroniques chez les animaux.
